# EUROPEAN PATENT APPLICATION

(11) **EP 1 393 738 A1**
(43) Date of publication of application: **03.03.2004**
(21) Application number: 02728123.7
(22) Date of filing: 23.05.2002
(51) Int. Cl.: A61K 35/84, A61K 31/716, A61P 3/10, A23L 1/30

(54) **DRUGS FOR DIABETES**

(30) Priority: 01.06.2001 JP 2001166919
(71) Applicant: Ajinomoto Co., Inc., Tokyo 104-8315 (JP)
(72) Inventor: MURATA, Yukie, Central Resch Lab Ajinomoto Co, Inc, Kawasaki-shi, Kanagawa 210-0801 (JP); HAMURO, Junji, Central Resch Lab Ajinomoto Co, Inc, Kawasaki-shi, Kanagawa 210-0801 (JP)
(74) Representative: HOFFMANN - EITLE
(86) International application number: PCT/JP2002/004981
(87) International publication number: WO 2002/098440

(57) **Abstract**

Provides a drug product for the prevention, improvement in conditions relating to, and/or treatment of diabetes, characterized by comprising an active component in the form of β (1→3) glucan derived from vegetable material and having a molecular weight of from 5,000 to 20,000.

Can be widely employed as a diabetes drug product for both IDDM and NIDDM, exhibits almost no side effects and does not have an obese effect even when orally administered, can be widely employed as a medical drug for patients in various states of morbidity, and can be employed by patients, generally healthy persons, and other animals in the form food and drink products such as therapeutic foods and health foods.

Also provides a method of preventing, improving conditions relating to, and/or treating diabetes and the use of the above-specified β (1→3) glucan in drug products.

## Description

### Technical Field

The present invention relates to a novel drug product specifically suited to the prevention, improvement in conditions relating to, and/or treatment of insulin-dependent diabetes mellitus (IDDM) and non-insulin-dependent diabetes mellitus (NIDDM). It may be administered orally, and due to a high degree of safety, employed in the form of food products such as health foods. The present invention further relates to a method of preventing, improving conditions relating to, and/or treating diabetes, and the use in drug products of the active component employed in this drug product for the prevention, improvement in conditions relating to, and/or treatment of diabetes.

### Prior Art

Diabetes is a chronic illness the main symptom of which is continuous high blood glucose. The number of patients suffering from diabetes is increasing worldwide, but there is no drug product that can be widely employed that is, for example, suited to numerous patients and safe, as well as desirably being effective when taken orally. Diabetes includes insulin-dependent diabetes mellitus (IDDM) and non-insulin-dependent diabetes mellitus (NIDDM). IDDM is a disease, often seen in youth, that tends to become life-threatening by deteriorating into hyperketosis for which there is no effective drug product and which is currently handled by administering insulin. Further, when NIDDM is left untreated, there is a risk of developing severe complications; early treatment is necessary. However, some patients upon which post-meal blood glucose. ameliorating agents have no effect are encountered, and there are no drug products that can be widely and conveniently employed in large numbers of patients due to side-effects and the inducing of obesity. In particular, the development of drug products that, with the onset of diabetes, prevent the occurrence or development of complications is an important issue and goal in the treatment of diabetes.

### Problems to Be Solved by the Invention

Given the above-described situation, there is a need for a drug product for diabetes patients, or high-risk patients, that can be widely employed for both IDDM and NIDDM, has almost no side-effects, produces no effects such as obesity, is effective when orally administered, and can be conveniently used by numerous patients. The problem solved by the present invention is the development of such a drug product.

### Disclosure of the Invention

The present inventors conducted extensive research into solving the above-stated problem. They discovered that in model animal experiments on mice given drinking water containing β (1→3) glucan with a specific molecular weight derived from vegetable material, the above-described desirable pharmacological effects were achieved. They discovered that this specific glucan could be used as a drug product for preventing, improving conditions relating to, and treating diabetes. And they discovered that this drug product could be specifically employed as a medical drug, be orally administered, could be consumed at meals by persons requiring treatment due to its good safety, and could be applied to, for example, healthy individuals in the form of a food or drink such as a health food product with the goal of prevention or improvement. The present invention was devised on the basis of these discoveries.

That is, the present invention is a drug product for the prevention, improvement in conditions relating to, and/or treatment of diabetes, characterized by comprising an active component in the form of β (1→3) glucan derived from vegetable material and having a molecular weight of from 5,000 to 20,000.

A further form of the present invention is a method of preventing, improving conditions relating to, and/or treating diabetes characterized in that β (1→3) glucan derived from vegetable material and having a molecular weight of from 5,000 to 20,000 is ingested by or administered to the body. The various above-described forms of the drug of the present invention may be employed in this administration or ingestion.

And a still further form of the present invention is the use of β (1→3) glucan derived from vegetable material and having a molecular weight of from 5,000 to 20,000 as a drug product for the prevention, improvement in conditions relating to, and/or treatment of diabetes. The various above-described forms of the drug product of the present invention may be employed as the drug product used in the prevention, improvement in conditions relating to, and/or treatment of diabetes.

### Brief Description of the Figures

[Fig. 1]

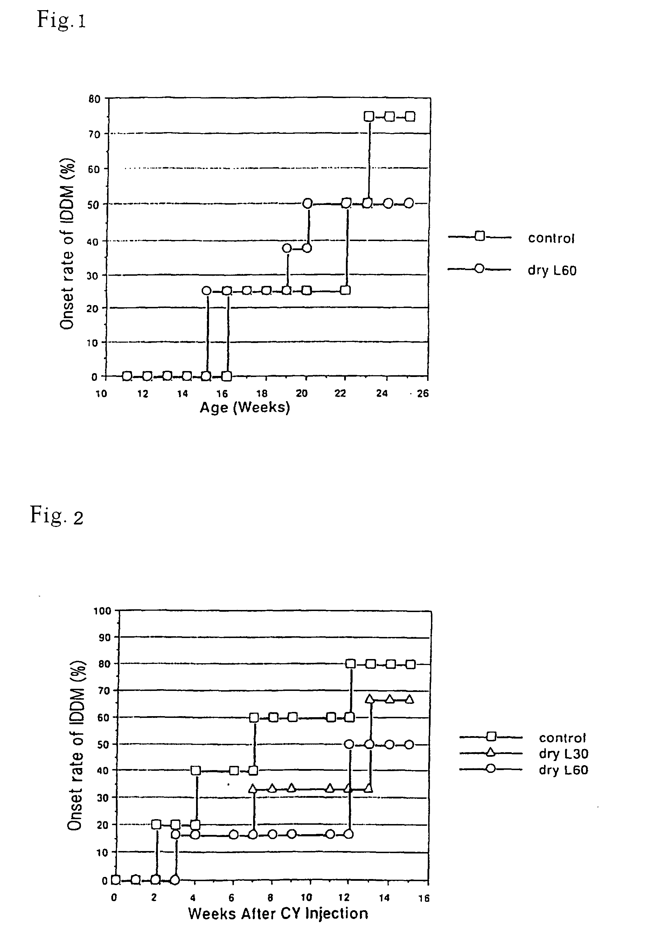

   Fig. 1 is a plot of the relation of administration during the Langerhans islet destruction period and the onset rate (%) of IDDM in Embodiment 1.
   □ : Control; ○ : Dry L60-min formic acid decomposed product.
[Fig. 2]
   Fig. 2 is a plot of the relation between dosing and IDDM onset rate (%) during, the insulitis stage in Embodiment 1.
   X-axis: Weeks after administration of cyclophosphamide (CY).
   □ : Control; Δ: Dry L30-min formic acid decomposed product; ○ : Dry L60-min formic acid decomposed product.
[Fig. 3]

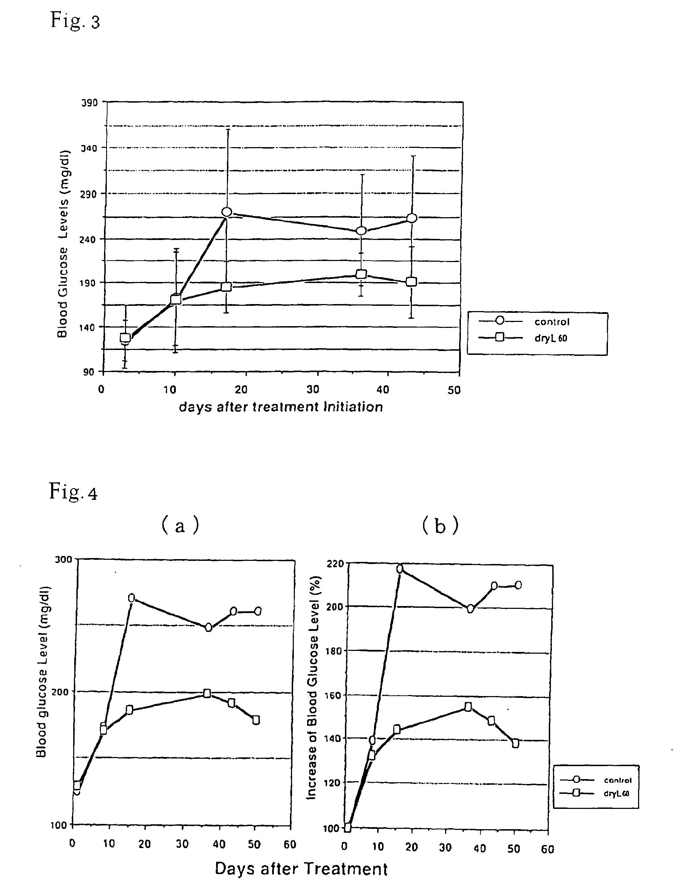

   Fig. 3 shows the suppressive effect on blood glucose level in Embodiment 2.
   X-axis: Number of days after treatment initiation.
   db/db mice (male), 5 weeks, n=6: ○ : control; □ : Dry L60-min formic acid decomposed product.
[Fig. 4]
   Fig. 4 shows the suppressive effect on blood glucose level in Embodiment 2. Fig. 4a: the relation to blood glucose level (mg/dL); Fig. 4b: the relation to the rate of increase in blood glucose level.
   X-axis: days after treatment initiation.
   db/db mice (male), 5 weeks, n=6: ○ : control; □ : Dry L60-min formic acid decomposed product.
[Fig. 5]

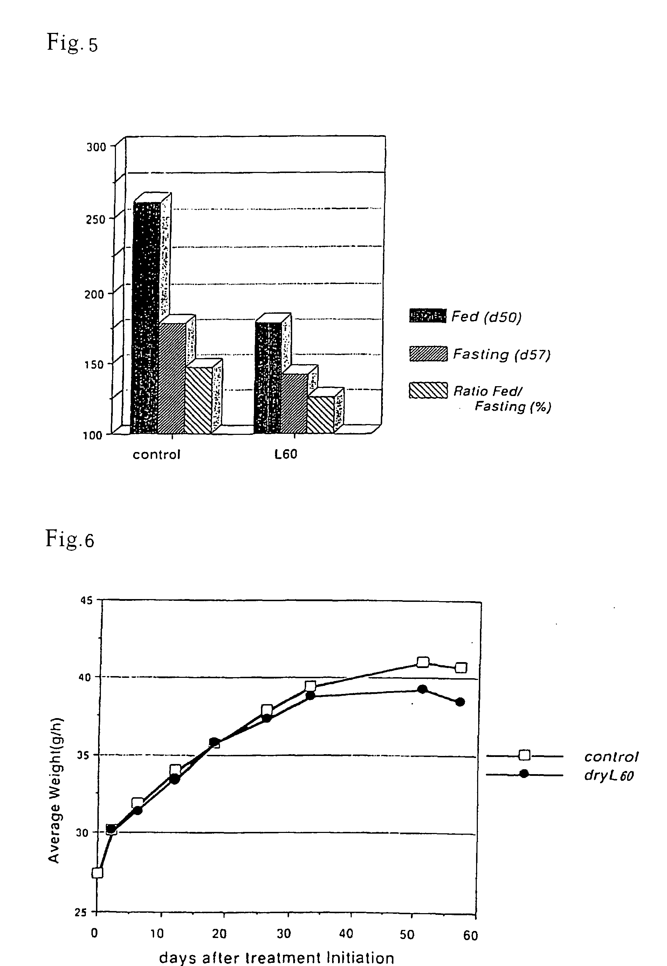

   Fig. 5 shows the suppressive effect on blood glucose level during feeding and fasting in Embodiment 2.
   Y-axis: ratio (%) of blood glucose level (mg/dL) when fed and when fasting; X-axis: from left, control and Dry L60-min formic acid decomposed product.
   Fed: fed; Fasting: fasting; d50 and d57: days after treatment initiation.
[Fig. 6]
   Fig. 6 shows the change in weight in Embodiment 2.
   Y-axis: weight per animal (g/h); X-axis: days after treatment initiation.
   db/db mice (male), 5 weeks, n=6: □ : Control; ● : Dry L60-min formic acid decomposed product.

### Modes of Implementing the Invention

Modes of implementing the invention are described below.

The application of the drug product of the present invention is not specifically limited beyond the prevention, improvement in conditions relating to, and treatment of diabetic disease, IDDM and NIDDM. As a medical drug, it is applied to mammals, usually humans (patients), and as a food or beverage, to healthy persons and patients seeking prevention or improvement.

The active component employed in the drug product of the present invention is β (1→3) glucan derived from vegetable material and having a molecular weight of from 5,000 to 20,000. The use of a component having a molecular weight denoted as an average molecular weight of from 5,000-20,000 as the principal component is convenient. To prepare a glucan with a molecular weight falling within this range, a glucan of relatively high molecular weight that has been hydrolyzed to obtain a molecule of low molecular weight ― for example, degradated with an enzyme such as β-(1-3) glucanase, chemically decomposed with formic acid or the like, or degradated through the use of a physical method ― is employed.

In the present invention, the term "β (1→3) glucan" includes all glucans having a β (1→3) bond, as well as glucans having a main chain in the form of a β (1→3) glusoside.

β (1→3) glucans obtained from mushrooms such as Matsutake [*Tricholoma matsudake*], Shiitake [*Lentinus edodes*], Bukuryo [*Poria cocos*], Kawaratake [*Coriolus versicolor*], Enokidake *[Flammulina veltipes*], Hiratake *[Pleurotus ostreatus*], Yamabushitake [*Hericium erinaceum*], and Agarikusuku [*Agaricus blazei murrill*] can be employed as the β (1→3) glucan derived from vegetable material (See Sasaki et al., Gann, 67, 191-195, April, 1976.). Such components can be readily prepared from mushrooms by, for example, obtaining an aqueous (hot water) extract, followed by precipitating with an alcohol (ethanol or the like) and, when needed, reducing the molecular weight thereof. Examples of methods for obtaining a molecular weight falling within the above-stated range are hydrolysis by suitable methods (enzymatic decomposition, hydrolysis with an acid such as formic acid, and decomposition by physical methods).

One of characteristics of the drug product of the present invention is orally effective and also can be applied to IDDM (non-insulin dependent diabetes mellitus). Safety is afforded, and there is no effect with regard to obesity. Accordingly, the form of administration is not specifically limited. Various forms of administration are possible, including oral administration, non-oral administration (intravenous administration and the like) are possible. Convenient, broad prevention and treatment is possible in diabetics and high risk patients.

As set forth above, the active component employed in the drug product of the present invention is highly safe and is suited to oral administration, permitting application in the form of health food products to prevention and improvement for such diseases. Further, the drug product of the present invention can be applied not just to the treatment of patients, but also in the form of food and drink products such as health food products to healthy persons in a preventive and ameliorative fashion.

The present invention also permits mixing and combining with other drug product components (pharmaceutically active substances). In such cases, so long as the active component of the present invention is present and the above-described targeted pharmacological activity is exhibited, the product is covered by the drug product of the present invention.

The incorporation of various substances that are pharmacologically acceptable in formulations (adjuvants and the like) is also possible. Formulation-use substances may be suitably selected based on the type of formulation. Examples are excipients, diluting agents, additives, anticaking agents, binders, coatings, lubricants, slipping agents, gloss-imparting agents, flavoring agents, sweetening agents, and solubilizing agents. Specific examples of formulation-use substances are magnesium carbonate, titanium dioxide, lactose, mannitol, other sugars, talc, milk protein, gelatin, starch, cellulose and its derivatives, animal and plant oils, polyethylene glycol, and solvents such as sterile water and monohydric and polyhydric alcohols such as glycerol.

The drug product of the present invention can be prepared in the above-described known forms as well as various medical drug formulations to be discovered in the future for, for example, oral administration, intraperitoneal administration, cutaneous administration, and inhalation. Known methods and methods developed in the future can be suitably employed to prepare various types of medical drug formulations of the drug product of the present invention.

Examples of these types of medical drug formulations are suitable solid and liquid formulations, such as grains, powders, coated tablets, tablets, (micro) capsules, suppositories, syrups, juices, suspensions, emulsions, titrations, injection solutions, and formulations affording extended release of active substances.

It is necessary that the above-described component be incorporated in a quantity suitably large to exhibit its drug effect in the formulation of the present invention based on the above-listed formulations.

The dosage of the drug product of the present invention is suitably selected based on the degree of severity of symptoms presented by the diabetic patient, the absence or presence, degree, and type of complications, the type of formulation, and the like. For example, in oral administration, based on the net weight of the active component, a daily dosage per patient of about 10 mg to 10 g is desirable, about 30 mg to 3 g is preferred, and about 500 mg to 2 g is even more preferred. In severe cases, even larger doses are possible. In terms of administration frequency and intervals, one administration every few days or one administration a day are both possible. Usually, however, there are several administrations per day, perhaps divided into 2 to 4 administrations, preferably before meals. Further, when administered intravenously, a dosage of about one-tenth to one-twentieth that of the above-described oral administration dosage is sufficient.

The drug product of the present invention can be broadly applied preventively and amelioratively to healthy persons, and to combat diabetes in patients who have already contracted the disease as a health food product, medical food product, or special health food product to provide meals. When employed as a health food product or the like, the above-described orally administered formulation can be referred to and orally administrable components and additives required by health food products can be added to the preparation. In that case, the drug product of the present invention can be provided in the form of food products (including all items placed in the mouth and chewed, such as chewing gum, teeth paste), nutrition agents, infusion formulations, and the like. These are also covered by the use of the drug product of the present invention. Medical food products may be in any form, including solids and liquids.

As set forth above, a further mode of the present invention is a method of preventing, improving conditions relating to, and/or treating diabetes characterized in that β (1→3) glucan derived from vegetable material and having a molecular weight of from 5,000 to 20,000 is ingested by or administered to the body, and a still further mode is the use of β (1→3) glucan derived from vegetable material and having a molecular weight of from 5,000 to 20,000 as a drug product for the prevention, improvement in conditions relating to, and/or treatment of diabetes.

All of these modes of the present invention, including their particular ingestion, administration, and use forms, can be readily practiced based on the above-described description of the drug product of the present invention, the embodiments described further below, and, as necessary, with reference to prior art.

### Desirable Modes of Implementing the Invention

The present invention is described in detail below based on embodiments. However, the present invention is not limited to the embodiments. The percentages employed in the embodiments are weight percentages unless specifically stated otherwise. (Samples and Laboratory Animals)
1) NOD mice (prepared by Clea Japan, Inc.), female;
2) NOD mouse feed ("CL-2, 30 Gy" powder, made by Clea Japan, Inc.);
3) db/db mice (prepared by Clea Japan, Inc.), male; and,
4) db/db mouse feed ("CRF-1 powder", made by Clea Japan, Inc.).

### (Preparation of Mushroom Product Extracted with Hot Water and Decomposed with Formic Acid)

### 1) 60-Minute Hydrolysis Product

To a round-bottom three-necked flask equipped with reflux condenser, temperature gauge, and mechanical stirrer were weighed out and charged 31.0 g of product (Dry L) obtained by hot-water extraction of raw Shiitake followed by precipitation from alcohol, 620 mL of 80 % formic acid was added, and the mixture was placed with being stirred in an oil bath preheated to 100°C. At 20 min, the temperature of the reaction solution reached 90°C, and stirring was continued for another 60 min. Subsequently, the reaction vessel was placed in an ice water bath to stop the reaction. The reaction solution was cooled to room temperature, yielding a brown gel-like substance. This was transferred to a two-liter eggplant-shaped flask. The solvent was distilled off under vacuum, yielding 50.4 g of a glue-like solid. To this were added two liters of pure water, the mixture was heated in a water bath to 60°C, the solid was dissolved to the extent possible, the mixture was left standing, and the clear supernatant was separated by decantation. To the undissolved portion in which was present a fibrous solid were added 800 mL of pure water and a household blender was employed to mix and pulverize for 60 min. To this was added the above-mentioned supernatant and the mixture was processed in an ultrasonic bath with ultrasound, yielding a uniform suspension. The suspension obtained was rapidly frozen in a dry ice ― alcohol bath and then freeze-dried, yielding 30.4 g of solid. A 0.22 % (by weight) suspension of this solid had a pH of 4.51. The average molecular weight was 12,200.

The Dry L consisted of 1 kg of raw Shiitake (*Lentinus edodes*) extracted in 5,000 mL of hot water and then precipitated (320 g) from 5,000 mL of ethanol. 2) 30-Min Hydrolysis Product

The same operation as above was conducted with the exception that the period of heating after reaching 90°C was shortened to 30 min in the above-described method of preparing a 60 min hydrolysis product, yielding 30.8 g of solid. The 0.22 % (by weight) suspension thereof had pH 4.86. The average molecular weight was 25,000.

### (Preparation of a 0.02 Weight Percent Aqueous Solution of the Dry L Hydrolysis Product)

200 mL quantities of the above-described Dry L60-min formic acid hydrolysis product or the Dry L30-min formic acid hydrolysis product were dissolved in each one-liter of sterile water (double distilled, followed by filtration with a millipore filter) and the pH values thereof for each were measured. The pH values were then adjusted to close to pH 7.0 with 1 normal sodium hydroxide aqueous solution to prepare Dry L 0.02 weight % aqueous solutions (a 0.02 weight % Dry L60-min formic acid decomposed product aqueous solution and a 0.02 weight % Dry L30-min formic acid decomposed product aqueous solution).

### (Preparation of Enzymatic Decomposition Product of Hot-Water Extract of Mushroom)

To a round-bottom three-necked flask equipped with reflux condenser, temperature gauge, and mechanical stirrer were weighed out and charged 30.0 g of raw Shiitake hot-water extract, the extract was dissolved in 2 L of sterile water, commercial β-(1-3) glucanase was added, stirring was conducted at 30°C, and the mixture was reacted. The reaction was continued for 60 min. Subsequently, the reaction vessel was immersed in an icewater bath to stop the reaction. The reaction solution was transferred to a five-liter eggplant-shaped flask, the solvent was distilled off under vacuum, and distillation was stopped before solid product precipitated. The clear supernatant was separated and the product was processed in an ultrasonic bath with ultrasound, yielding a clear solution. The solution obtained was rapidly frozen in a dry ice ― alcohol bath and freeze dried, yielding 32.4 g of solid product. The 0.22 % (by weight) suspension of this solid had a pH of 6.52. The average molecular weight was 9,200.

### (Inducement of IDDM by Administration of Cyclophosphamide (CY) to NOD Mice)

Disease was induced in 11 week NOD female mice by one administration of 0.15 mL (150 mg/kg) cyclophosphamide ("Endoxan" made by Shionogi Seiyaku) per mouse prepared to 20 mg/mL with infusion-use distilled water (made by Otsuka Seiyaku)into the abdominal cavity wherein a 25 G needle (made by Termo Co.) with a 1 mL syringe was used.

### (Determination of Onset of IDDM in NOD Mice).

Onset was determined by the detection of glucose in urine using a BM Test Glucose 5000 made by Yamanouchi Seiyaku once a week from the first week after the administration of cyclophosphamide.

### (Method of Measuring the db/db Mouse Blood Glucose Level)

db/db mice naturally contract obese-type NIDDM. Blood sugar level measurement was conducted to determine the degree of NIDDM onset.

A db/db mouse was driven into work gloves and a wound was made in a vein with a razor (made by Kaijirushi) about 1 cm from the front end of the tail of the mouse. About 6 µL of blood were collected with a pipette from the wound and suspended (twofold dilution) in 6 µL of physiological saline (made by Otsuka Seiyaku) prepared in advance. A 6 mL quantity was added onto a Fuji DryChem slide GLU-W (made by Fuji Photographic Film) that had been set on a Fuji DryChem 5000 (made by Fuji Photographic Film), light absorbance at 505 nm was measured, and the concentration (mg/dL) was calculated.

### (Embodiment 1)

### Testing the Effect of Orally Ingested Dry L Hydrolysis Product Using an IDDM Model

The intake of Dry L hydrolysis product during the period of inflammation of pancreatic islets (insulitis) due to the administration of cyclophosphamide suppressed the onset of diabetes. A specific description is given below.

NOD mice are known as IDDM onset model animals. Insulitis occurs in NOD mice at about 4 to 6 weeks of age. The Langerhans islets of the pancreas are damaged through a cellular immunological mechanism at 14 to 18 weeks, causing a failure to produce insulin and thus causing the onset of IDDM in NOD mice. Two different morbid states are thought to be found in varying tissue inflammation states. Accordingly, during the period of insulitis and during the period of damage to the Langerhans islets, aqueous solutions (aqueous solutions of Dry L formic acid decomposed products) of Dry L hydrolysis decomposed product were provided as drinking water and the resulting IDDM onset rate was compared with that of a control group.
a) Even when an aqueous solution of Dry L hydrolysis product was ingested orally during the preliminary Langerhans islet destruction period by NOD mice, no suppression of onset was observed. A specific description is given below.

A 0.02 weight % Dry L60-min formic acid decomposed product aqueous solution was provided as drinking water at age 12-16 weeks to NOD female mice. The control group was given sterile water. The natural onset rates of the two were compared. At 25 weeks of age, the onset rate of the control group was 75 %, while that of the group drinking 0.02 weight % Dry L60-min formic acid decomposed product aqueous solution was 50 %. A significant difference was thus not observed between the onset rates of the two (see Fig. 1). This shows that even when an aqueous solution of Dry L hydrolysis product is orally ingested during the Langerhans islet destruction period, no suppression of onset is observed.
b) When an aqueous solution of Dry L hydrolysis product was orally administered to NOD mice during the insulitis period, onset was suppressed. A specific description is given below.

From age 4 to 14 weeks, female NOD mice were given drinking water containing 0.02 weight % Dry L60-min formic acid decomposed product or 0.02 weight % Dry L30-min formic acid decomposed product. The control group was given sterile water as drinking water. At the age of 11 weeks, cyclophosphamide was administered to induce IDDM and the rates of onset of the two were compared.

The rate of onset at week 7 following the administration of cyclophosphamide was 60 % in the control group, 16.6 % in the group that had been given drinking water containing 0.02 weight % Dry L60-min formic acid decomposed production, and 35 % in the group that had been given drinking water containing Dry L30-min formic acid decomposed product (see Fig. 2).

Significant suppression of onset was seen in the group given drinking water containing 0.02 weight % Dry L60-min formic acid decomposed production, confirming that the oral ingestion of an aqueous solution of Dry L hydrolytic product during the insulitis period had a suppressive effect on IDDM onset in NOD mice.

### (Embodiment 2)

### Testing the Effect of Orally Ingested Dry L Using an NIDDM Model

The oral intake of Dry L hydrolysis product suppressed the progression of the morbid state of NIDDM.

db/db mice are known as a model of the morbid state of obese-type NIDDM. With the progression of NIDDM, a rise in the level of blood glucose is observed. Using the blood glucose level and body weight as indicators of the progression of morbidity, db/db mice were given drinking water containing 0.02 weight % Dry L60-min formic acid decomposed product, the progression of the state of morbidity of this group was compared with that of a control group given sterile water as drinking water, and the results of oral ingestion of Dry L were examined.

Male db/db mice were given drinking water containing Dry L60-min formic acid decomposed product from age 5 weeks to 13 weeks. The blood glucose level was measured once a week during feeding or during fasting with a Fuji DryChem (fasting was conducted from the afternoon of the day preceding measurement to the time of measurement).

From day 10 after the start of ingestion of the drinking water, the group ingesting drinking water containing Dry L60-min formic acid decomposed product had lower blood glucose levels than the control group, both when fed and when fasting. A significant increase in the suppression of blood glucose levels was observed in the group ingesting drinking water containing Dry L60-min formic acid decomposed product. A comparison of the maximum level of blood glucose of each group during the period of ingestion of drinking water with the blood glucose level at the start of ingestion of drinking water (that is, the increase in blood glucose relative to the start of the test) was 120 % for the control group and 50 % for the group ingesting drinking water containing Dry L60-min formic acid decomposed product; a significant increase in the suppression of blood glucose level was confirmed. This resulting suppression of the increase in blood glucose level continued for three weeks following the termination of ingestion of the drinking water. No difference was observed in the quantity of feed consumed between the two during that time. Further, the group ingesting drinking water containing Dry L60-min formic acid decomposed product exhibited a significantly lower level of increase in body weight relative to the control group from day 30 following the start of ingestion of the drinking water (see Figs. 3 to 6).

The above results suggested that ingesting drinking watercontaining Dry L60-min formic acid decomposed product suppressed the progression of the morbid state of NIDDM.

### (Embodiment 3)

### Testing the Effect of Orally Ingested Enzymatic Decomposition Product Using an NIDDM Model

The oral ingestion of an enzymatic decomposition product of Shiitake hot-water extract suppressed the development of the morbid state of NIDDM.

Employing blood glucose level and body weight as indicators of the progression of morbidity, db/db mice were given drinking water containing 0.01 weight % of Shiitake hot-water extract that had been enzymatically broken down. The progression of morbidity was compared to that of a control group given drinking water in the form of sterile water, and the results of the suppression of the onset of NIDDM by oral ingestion were examined.

Male db/db mice were given drinking water containing enzymatically degradated product from 5 weeks to 13 weeks of age, and blood glucose was measured once a week when fed and when fasting with a Fuji DryChem (fasting was conducted from the afternoon of the day preceding measurement to the time of measurement).

From day 10 after the start of ingestion of the drinking water, the group ingesting drinking water in the form of an aqueous solution of enzymatically decomposed product exhibited lower blood glucose levels than the control group, both when fed and when fasting. Significant suppression of blood glucose was confirmed in the group ingesting drinking water containing enzymatically decomposed product. A comparison of the maximum level of blood glucose of each group during the period of ingestion of drinking water with the blood glucose level at the start of ingestion of drinking water (that is, the increase in blood glucose relative to the start of the test) was 140 % for the control group and 38 % for the group ingesting drinking water containing enzymatically decomposed product; a significant increase in the suppression of blood glucose level was confirmed. This resulting suppression of the increase in blood glucose level continued for four weeks following the termination of administration. No difference was observed in the quantity of feed consumed between the two groups during that time.

From the above results, it will be understood that the ingestion of drinking water containing enzymatically decomposed product significantly suppressed the morbid progression of NIDDM relative to the control group.

### Effect of the Invention

The present invention provides a drug product that can be widely used on states of both IDDM and NIDDM diabetic morbidity, has almost no side effects, has no obese effect, and is effective even when orally administered, thus making it suitable as a medical drug product for widespread and convention use by patients in various morbid states, in the prevention, improvement in conditions relating to, treatment, and the like of diabetes.

In addition to being provided as a medical drug for combating diabetes, the drug product of the present invention can be provided for use as a food and drink product to healthy persons for prevention and improvement in conditions relating to, to suppress the further progression of diabetes, and as a food product to patients suffering from such conditions. Accordingly, the drug product of the present invention can be provided in the form of health food products, therapeutic food products, and the like. Still further, the present invention provides a method of preventing, improving conditions relating to, and/or treating diabetes, and the use of specific β(1→3) glucan in such drug products.

Accordingly, the present invention can be widely implemented in the fields of medical drugs, food products, medical treatment, feeds, and veterinary drugs, and is thus extremely useful from an industrial perspective.

## Claims

1. A drug product for the prevention, improvement in conditions relating to, and/or treatment of diabetes, **characterized by** comprising an active component in the form of β (1→3) glucan derived from vegetable material and having a molecular weight of from 5,000 to 20,000.

2. The drug product of claim 1 wherein said vegetable matter is a mushroom.

3. The drug product of claim 1 or 2 wherein said β (1→3) glucan is obtained by hydrolysis of a glucan.

4. The drug product of any of claims 1 to 3 wherein said β (1→3) glucan is obtained from the aqueous extraction product of a mushroom.

5. The drug product of claim 4 wherein said glucan obtained from the aqueous extraction product of a mushroom is obtained by the aqueous extraction ― alcohol precipitation of a mushroom or the further decomposition of the precipitate to obtain a compound of low molecular weight.

6. The drug product of any of claims 1 to 4 that is administered orally.

7. The drug product of claim 1 where said diabetes is insulin-dependent or non-insulin dependent diabetes mellitus.

8. The drug product of claim 1 where said molecular weight of from 5,000 to 20,000 denotes an average molecular weight of from 5,000 to 20,000.

9. The drug product of claim 2 wherein the type of mushroom is Shiitake.

10. The drug product of claim 1 in the form of a health food product.

11. A method of preventing, improving conditions relating to, and/or treating diabetes **characterized in that** β (1→3) glucan derived from vegetable material and having a molecular weight of from 5,000 to 20,000 is ingested by or administered to the body.

12. The method of claim 11 wherein the form that is ingested or administered is that of the drug product described in any of claims 1 to 10.

13. The use of β (1→3) glucan derived from vegetable material and having a molecular weight of from 5,000 to 20,000 as a drug product for the prevention, improvement in conditions relating to, and/or treatment of diabetes.

14. The use of the drug product described in claim 13 in the form of the any of the drug products described in claims 1 to 10.
